# EUROPEAN PATENT APPLICATION

(11) **EP 3 919 027 A1**
(43) Date of publication of application: **08.12.2021**
(21) Application number: 19913425.5
(22) Date of filing: 23.12.2019
(51) Int. Cl.: A61F 2/38

(54) **UNICONDYLAR FEMORAL PROSTHESIS, TIBIA PAD, AND UNICONDYLAR REPLACEMENT PROSTHESIS**

(30) Priority: 31.01.2019 CN 201910098530
(71) Applicant: Suzhou Microport Orthorecon Co., Ltd., Suzhou, Jiangsu 215000 (CN)
(72) Inventor: SUN, Yandong, Suzhou, Jiangsu 215000 (CN); ZHAO, Kaiyu, Suzhou, Jiangsu 215000 (CN); SHI, Zhongbing, Suzhou, Jiangsu 215000 (CN)
(74) Representative: Dauncey, Mark Peter
(86) International application number: PCT/CN2019/127558
(87) International publication number: WO 2020/155932

(57) **Abstract**

A unicondylar femoral prosthesis (100, 200, 200), a tibia pad (400), and a unicondylar replacement prosthesis. The unicondylar femoral prosthesis (100, 200, 300) comprises an osteotomy surface (120) for connecting to the femur and a joint surface (110) for abutting the tibia, the joint surface (110) comprising a distal end joint surface (111) and a posterior condyle joint surface (112). The distal end joint surface (111) has a first radius of curvature (Rd) on the sagittal plane (101) and the posterior condyle joint surface (112) has a second radius of curvature (Rp) on the sagittal plane, the range of the ratio of the first radius of curvature (Rd) to the second radius of curvature (Rp) being 1.4-2.1. The joint surface (110) has a third radius of curvature (Rc) on the coronal plane (102), the third radius of curvature (Rc) being equal to the first radius of curvature (Rd). The present unicondylar femoral prosthesis (100) better matches the anatomical structure of the human femoral joint surface, avoiding the problems of the insufficient coverage of the femoral distal end surface and excessive osteotomy, and improving the bearing performance of the joint surface (110) and the joint stability.

## Description

### CROSS-REFERENCE TO RELATED APPLICATION

This application claims priority to Chinese Patent Application No. 201910098530.7, entitled "UNICONDYLAR FEMORAL PROSTHESIS AND TIBIAL PAD" and filed on January 31, 2019, the entire contents of which are incorporated herein by reference.

### TECHNICAL FIELD

The present application relates to the fields of medical equipment technologies, more particularly, to a unicondylar femoral prosthesis, a tibial pad and a unicondylar replacement prosthesis.

### BACKGROUND

Unicondylar replacement surgery has been widely used in the treatment of singlecompartment bone and joint due to its advantages of small wound and quick recovery. Types of unicondylar prostheses used in the unicondylar replacement surgery may include a medial femoral prosthesis and a medial tibial prosthesis that are configured to replace a medial compartment, and a lateral femoral prosthesis and a lateral tibial prosthesis that are configured to replace a lateral compartment.

In order to ensure that a contact area between a femoral component and a tibial component remains unchanged throughout a flexion and extension range, an articular surface of a conventional unicondylar femoral prosthesis is mostly a single-radius spherical design. Since a shape of an articular surface of a posterior condyle of a femur is closer to a spherical surface, the single-radius spherical design mainly covers the posterior condyle of the femur; that is, the single-radius spherical design performs fitting based on the contour of the articular surface of the posterior condyle. Therefore, the single-radius spherical design cannot match a distal femur well. At the same time, a recessed region may be formed in the distal femur during osteotomy, resulting in excessive osteotomy at the distal femur. Since the distal femur is a main load-bearing surface of a joint during daily physiological activities, walking and standing, such mismatch may increase a risk of loosening of a femoral condyle prosthesis after implantation. In addition, some unicondylar femoral prostheses use a multi-center design with a gradually decreasing curvature radius from a distal articular surface to a posterior condyle articular surface. However, such femoral prostheses may loosen ligament tissues during flexion of a knee joint, resulting in decreased joint stability, poor postoperative proprioception, and a high risk of prosthetic dislocation.

### SUMMARY

Various embodiment disclosed in the present application provide a unicondylar femoral prosthesis, a tibial pad and a unicondylar replacement prosthesis.

One aspect of the present application provides a unicondylar femoral prosthesis, including an osteotomy surface configured to connect with a femur and an articular surface configured to abut a tibial pad, the articular surface including a distal articular surface and a posterior condyle articular surface, the distal articular surface having a first curvature radius on a sagittal plane, and the posterior condyle articular surface having a second curvature radius on the sagittal plane, wherein a ratio of the first curvature radius to the second curvature radius ranges from 1.4 to 2.1, the articular surface has a third curvature radius on a coronal plane, and the third curvature radius is equal to the first curvature radius.

The unicondylar femoral prosthesis uses an articular surface with double radii on the sagittal plane, that is, the distal articular surface with the first curvature radius and the posterior condyle articular surface with the second curvature radius on the sagittal plane, and the first curvature radius is greater than the second curvature radius, so that the unicondylar femoral prosthesis is better matched with the anatomical structure of the human femoral articular surface, which solves the problems of insufficient coverage and excessive osteotomy of the femoral distal surface by the conventional spherical single-radius prosthesis.

In one embodiment, the osteotomy surface includes a posterior condyle osteotomy surface, a posterior oblique osteotomy surface and a distal osteotomy surface that are sequentially connected, the posterior condyle osteotomy surface, the posterior oblique osteotomy surface and the distal osteotomy surfaces are all flat surfaces, and the posterior condyle osteotomy surface and the distal osteotomy surface are perpendicular to each other.

In one embodiment, the osteotomy surface includes a posterior condyle osteotomy surface and a distal osteotomy surface, the posterior condyle osteotomy surface is a flat surface, the distal osteotomy surface is a spherical surface, and the distal osteotomy surface is concentric with the distal articular surface.

In one embodiment, a curvature radius of the distal osteotomy surface is 4 mm to 7 mm less than the first curvature radius.

In one embodiment, a curved surface deviating from a direction of a knee joint center is formed on one side of the unicondylar femoral prosthesis close to the knee joint center.

In one embodiment, the curved surface extends from a point located on a first half part of one side of the unicondylar femoral prosthesis close to the knee joint center to a point located at an anterior end of the unicondylar femoral prosthesis.

In one embodiment, the osteotomy surface is provided with at least one fixing post for assisting the fixation of the unicondylar femoral prosthesis.

In one embodiment, a transition angle between the distal articular surface and the posterior condyle articular surface ranges from 10° to 25°. The transition angle is an angle formed, on the sagittal plane, by a straight line passing through a center of curvature of the distal articular surface and passing through a farthest point of the distal articular surface and a straight line passing through a center of curvature of the posterior condyle articular surface and passing through an intersection point between the distal articular surface and the posterior condyle articular surface.

In one embodiment, the unicondylar femoral prosthesis is a medial femoral prosthesis configured to replace a medial compartment of a femur, and a first curvature radius of the medial femoral prosthesis ranges from 30 mm to 55 mm.

In one embodiment, the medial femoral prosthesis has a lateral surface and a medial surface, and both the lateral surface and the medial surface include a curved surface curved toward the knee joint center.

In one embodiment, a projection of the lateral surface on a horizontal plane forms an arc, and an angle between a tangent at a midpoint of the arc and the sagittal plane ranges from 15° to 30°.

In one embodiment, the unicondylar femoral prosthesis is a lateral femoral prosthesis configured to replace a lateral compartment of a femur, and a first curvature radius of the lateral femoral prosthesis ranges from 30 mm to 60 mm.

In one embodiment, the lateral femoral prosthesis has a lateral surface and a medial surface, the lateral surface including a first straight surface and a second straight surface that are toward the knee joint center. The medial surface includes a third straight surface and a fourth straight surface that are toward the knee joint center.

In one embodiment, the first straight surface is parallel to the third straight surface, and an angle between the first straight surface or the third straight surface and the sagittal plane ranges from 0° to 6°; and the second straight surface is parallel to the fourth straight surface, and an angle between the second straight surface or the fourth straight surface and the sagittal plane ranges from 7° to 14°.

Another aspect of the present application provides a tibial pad for mating with the unicondylar femoral prosthesis described above, wherein the tibial pad has a proximal articular surface abutting the distal articular surface, the proximal articular surface is a spherical surface, and a ratio of the first curvature radius to a curvature radius of the proximal articular surface ranges from 0.95 to 1.

The tibial pad can be matched with the unicondylar femoral prosthesis, thereby reducing the wear of the unicondylar femoral prosthesis and prolonging the service life of the unicondylar femoral prosthesis.

Still another aspect of the present application provides a unicondylar replacement prosthesis, including the unicondylar femoral prosthesis according to any one of the above embodiments and the tibial pad according to any one of the above embodiments.

### BRIEF DESCRIPTION OF THE DRAWINGS

Figure 1 is a schematic diagram of various anatomical surfaces of a human body;
Figure 2 is a view of a sagittal plane of a knee joint;
Figure 3 is a view of a coronal plane of a knee joint;
Figure 4 is a side view of a unicondylar femoral prosthesis according to an embodiment;
Figure 5 is a top view of the unicondylar femoral prosthesis shown in Figure 4;
Figure 6 is a side view of a unicondylar femoral prosthesis according to another embodiment;
Figure 7 is a top view of the unicondylar femoral prosthesis shown in Figure 6;
Figure 8 is a side view of a medial femoral prosthesis according to an embodiment;
Figure 9 is a top view of the medial femoral prosthesis shown in Figure 8;
Figure 10 is a schematic diagram of mounting of the medial femoral prosthesis shown in Figure 8;
Figure 11 is a side view of a lateral femoral prosthesis according to an embodiment;
Figure 12 is a top view of the lateral femoral prosthesis shown in Figure 11;
Figure 13 is a schematic diagram of mounting of the lateral femoral prosthesis shown in Figure 11;
Figure 14 is a top view of a medial femoral prosthesis according to another embodiment;
Figure 15 is a top view of a lateral femoral prosthesis according to another embodiment;
Figure 16 is a schematic diagram of a tibial prosthesis according to an embodiment; and
Figure 17 is a cross-sectional view of the tibial prosthesis shown in Fig. 16 along an A-A section.

### DETAILED DESCRIPTION OF THE EMBODIMENTS

In order to make the above objectives, features and advantages of the present application more obvious and understandable, specific implementations of the present application are described in detail below with reference to the accompanying drawings. In the following description, many specific details are set forth in order to fully understand the present application. However, the present application can be implemented in many other ways different from those described herein, and those skilled in the art can make similar improvements without departing from the connotation of the present application. Therefore, the present application is not limited by the specific implementation disclosed below.

It should be noted that when one element is referred to as "fixed to" another element, it may be directly on the other element or an intermediate element may exist. When one element is considered to be "connected to" another element, it may be directly connected to the other element or an intermediate element may co-exist.

Unless defined otherwise, all technical and scientific terms used herein have the same meanings as are commonly understood by those skilled in the art. The terms used herein in the specification of the present application are for the purpose of describing specific embodiments only but not intended to limit the present application.

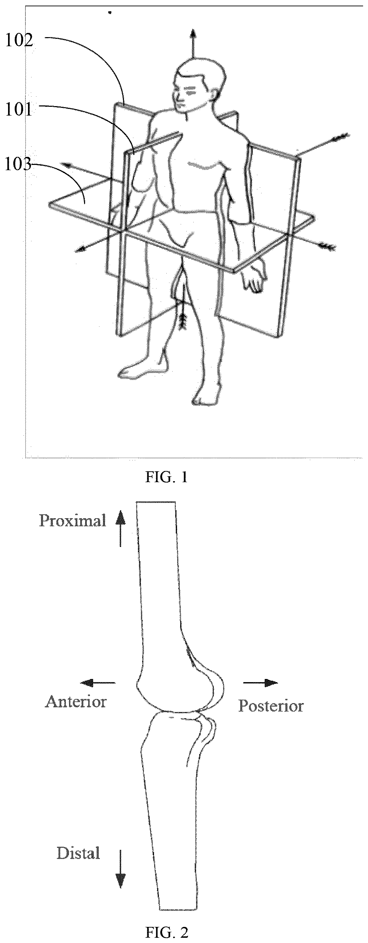

In order to better explain the technical solutions of the present application, firstly, orientation names involved in each embodiment are explained, as shown in Figures 1-3.

Sagittal plane 101: it refers to a longitudinal section that divides a human body or joint into left and right parts from an anterior-posterior direction. A sagittal plane passing through the middle of the human body is a median sagittal plane which divides the human body into left and right parts equal to each other.

Coronal plane 102: it refers to a longitudinal section that divides the human body or joint into anterior and posterior parts from a left-right direction. The section is perpendicular to the sagittal plane.

Horizontal plane 103: it is also known as a transverse section, and is a plane parallel to the ground plane and dividing the human body or joint into upper and lower parts. The plane is perpendicular to the coronal plane and the sagittal plane.

Distal: the end of the human body or joint relatively far away from the head.

Proximal: the end of the human body or joint relatively close to the head.

Medial: the side relatively close to the median sagittal plane of the human body.

Lateral: the side relatively far away from the median sagittal plane of the human body.

Anterior: the side relatively close to the abdomen in the sagittal plane.

Posterior: the side relatively close to the back in the sagittal plane.

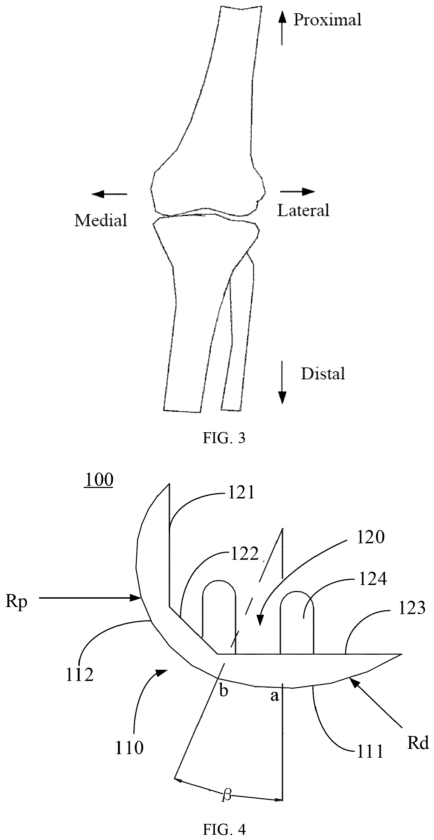

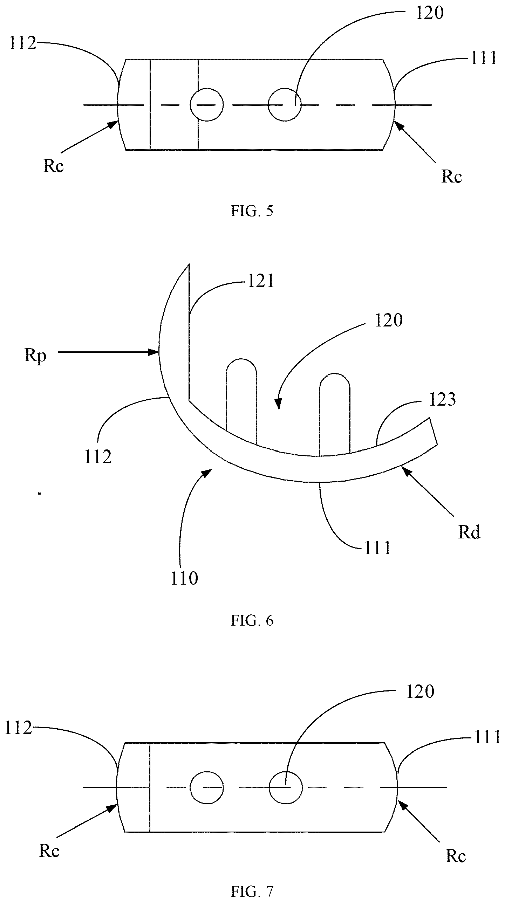

Referring to Figure 4 and Figure 5, each exemplary embodiment of the present application provides a unicondylar femoral prosthesis 100 configured to replace a medial compartment or a lateral compartment of a knee joint. Specifically, the unicondylar femoral prosthesis 100 according to an embodiment includes an articular surface 110 and an osteotomy surface 120. The osteotomy surface 120 is configured to connect with a femur to fix the unicondylar femoral prosthesis 100. The articular surface 110 is configured to abut a tibia to replace a diseased femoral articular surface. The articular surface 110 is a curved surface protruding distally, and the articular surface 110 includes a distal articular surface 111 configured to replace a distal end of the femur and a posterior condyle articular surface 112 configured to replace a posterior condyle of the femur. The distal articular surface 111 has a first curvature radius Rd on a sagittal plane, and the posterior condyle articular surface 112 has a second curvature radius Rp on the sagittal plane. The first curvature radius Rd is greater than the second curvature radius Rp, so that the articular surface 110 forms a curved surface with double radii on the sagittal plane to better match the distal end and the posterior condyle of the femur. Further, the articular surface 110 has a third curvature radius Rc on a coronal plane, and the third curvature radius Rc is equal to the first curvature radius Rd, so that the distal articular surface 111 forms a spherical surface whose radius is the first curvature radius Rd. Since the spherical surface has better load-bearing performance, the distal articular surface 111, which is a main load-bearing surface in daily walking and standing, has better load-bearing capability.

The unicondylar femoral prosthesis 100 uses an articular surface 110 with double radii on the sagittal plane, that is, the distal articular surface 111 with the first curvature radius Rd and the posterior condyle articular surface 112 with the second curvature radius Rp on the sagittal plane. Since the distal articular surface 111 of the human femur has a greater curvature radius than the posterior condyle articular surface 112, the first curvature radius Rd is set to be greater than the second curvature radius Rp, so that the articular surface 110 is better matched with the anatomical structure of the human femoral articular surface, which solves the problems of insufficient coverage and excessive osteotomy of the femoral distal surface by the conventional spherical single-radius prosthesis. Further, the third curvature radius Rc of the articular surface 110 of the unicondylar femoral prosthesis 100 on the coronal plane is equal to the first curvature radius Rd, so that the distal articular surface 111 forms a spherical surface, avoiding the instability of flexion of the knee joint caused by the conventional multi-center femoral prosthesis, and improving the load-bearing performance of the distal articular surface 111 and the joint stability.

The osteotomy surface 120 of the unicondylar femoral prosthesis 100 matches a femoral osteotomy surface formed in the unicondylar replacement surgery. In one embodiment, the osteotomy surface 120 includes a posterior condyle osteotomy surface 121, a posterior oblique osteotomy surface 122, and a distal osteotomy surface 123 that are sequentially connected. The posterior condyle osteotomy surface 121, the posterior oblique osteotomy surface 122 and the distal osteotomy surface 123 are all flat surfaces, and the posterior condyle osteotomy surface 121 and the distal osteotomy surface 123 are perpendicular to each other. Further, angles among the posterior oblique osteotomy surface 122, the posterior condyle osteotomy surface 121 and the distal osteotomy surface 123 range from 130° to 140°, preferably 135°. The posterior condyle osteotomy surface 121, the posterior oblique osteotomy surface 122 and the distal osteotomy surface 123 are set to flat surfaces, which makes the processing of the osteotomy surface 120 simple and reduces the process difficulty.

Referring to Figure 6 and Figure 7, in another embodiment, the distal osteotomy surface 123 may also be replaced by a spherical surface. Specifically, the osteotomy surface 120 according to an embodiment includes a posterior condyle osteotomy surface 121 and a distal osteotomy surface 123. The posterior condyle osteotomy surface 121 is a flat surface. The distal osteotomy surface 123 is a spherical surface, and the distal osteotomy surface 123 is concentric with the distal articular surface 111; that is, a center of curvature of the distal osteotomy surface 123 coincides with a center of curvature of the distal articular surface 111. Further, the curvature radius of the distal osteotomy surface 123 is 4 mm to 7 mm less than the curvature radius of the distal articular surface 111, preferably 4.5 mm to 6.5 mm, more preferably 6 mm. Within this range, a thickness of the prosthesis can meet the requirements of mechanical strength, and the amount of osteotomy can be controlled as much as possible to prevent excessive osteotomy. It should be noted that all numerical ranges in this article include endpoint values. The spherical structure of the distal osteotomy surface 123 can retain more cortical bones of the femur, so that an original cancellous bone structure of the femur can be better maintained during osteotomy, the postoperative load-bearing performance of the femur is better, and the postoperative loosening probability of the unicondylar femoral prosthesis 100 is reduced.

In one embodiment, the osteotomy surface 120 of the unicondylar femoral prosthesis 100 is provided with at least one fixing post 124. Specifically, referring to Figure 4, the osteotomy surface 120 is provided with two fixing posts 124. The fixing post 124 is cylindrical and is configured to assist the fixation of the unicondylar femoral prosthesis 100. Specifically, in the replacement surgery of the unicondylar femoral prosthesis 100, a mounting hole is opened on the femoral osteotomy surface, and the fixing post 124 is arranged in the mounting hole, so as to achieve the purpose of fixing the unicondylar femoral prosthesis 100.

According to different replacement positions, types of the unicondylar femoral prosthesis 100 may include a medial femoral prosthesis configured to replace a medial femoral compartment and a lateral femoral prosthesis configured to replace a lateral femoral compartment. When the unicondylar femoral prosthesis 100 is a medial femoral prosthesis, the first curvature radius Rd ranges from 30 mm to 55 mm, preferably 36 mm to 46 mm, more preferably 41 mm. If the first curvature radius Rd is within this numerical range, the shape of the distal articular surface of the prosthesis is better matched with an original physiological structure of the distal articular surface of the femur of the human body. The ratio of the first curvature radius Rd to the second curvature radius Rp ranges from 1.4 to 2.1, preferably 1.6 to 2.0, more preferably 1.7, so that the medial femoral prosthesis better matches the medial femoral compartment. Further, the transition angle β between the distal articular surface 111 and the posterior condyle articular surface 112 ranges from 10° to 25°, preferably 15° to 20°, and more preferably 20°. The transition angle within this range can better balance the joint range of motion and friction performance. The transition angle β is defined as an angle formed, on the sagittal plane as shown in Figure 4, by a straight line passing through a center of curvature of the distal articular surface 111 and passing through a farthest point a of the distal articular surface 111 and a straight line passing through a center of curvature of the posterior condyle articular surface 112 and passing through an intersection point b between the distal articular surface 111 and the posterior condyle articular surface 112.

When the unicondylar femoral prosthesis 100 is a lateral femoral prosthesis, the first curvature radius Rd ranges from 30 mm to 60 mm, preferably 41 mm to 51 mm, more preferably 46 mm. If the first curvature radius Rd is within this numerical range, the shape of the distal articular surface of the prosthesis is better matched with an original physiological structure of the distal articular surface of the femur of the human body. The ratio of the first curvature radius Rd to the second curvature radius Rp ranges from 1.4 to 2.1, preferably 1.6 to 2.0, more preferably 1.7, so that the lateral femoral prosthesis better matches the lateral femoral compartment. Further, the transition angle β between the distal articular surface 111 and the posterior condyle articular surface 112 ranges from 10° to 25°, and the transition angle β within this range can better balance the joint range of motion and friction performance.

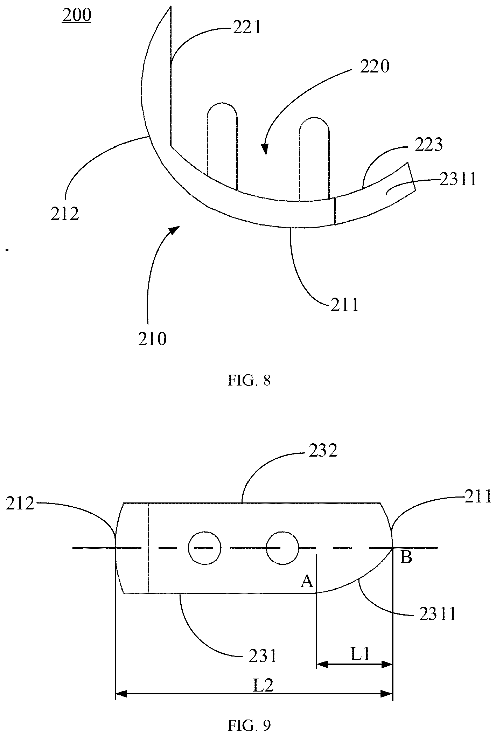

Exemplary embodiments of the present application also provide an asymmetric unicondylar femoral prosthesis. Specifically, referring to Figures 8-10, taking the medial femoral prosthesis 200 of the right knee joint as an example, the side of the medial femoral prosthesis 200 close to a knee joint center 500 is a medial surface 231 of the medial femoral prosthesis 200, and the side of the medial femoral prosthesis 200 away from the knee joint center 500 is a lateral surface 232 of the medial femoral prosthesis 200. A curved surface 2311 deviating from a direction of the knee joint center 500 is formed on the medial surface 231 of the medial femoral prosthesis 200. Specifically, in the top view shown in Figure 9, the curved surface 2311 extends from a point A located on a first half part of the medial surface 231 of the medial femoral prosthesis 200 to a point B located at an anterior end of the medial femoral prosthesis 200. Preferably, the point A is located at the first 35% to 45% of the medial surface 231. More preferably, the point A is located at the first 40% of the medial surface 231. "Located at the first 35% to 45% of the medial surface 231" means that a distance L1 from the point A to the point B in an anterior-posterior direction accounts for 35% to 45% of a distance L2 from the most anterior end to the most posterior end of the medial femoral prosthesis 200 in the anterior-posterior direction. Preferably, the point B is located at a midpoint of the anterior end of the medial femoral prosthesis 200. The medial femoral prosthesis 200 with the curved surface 2311 can reduce the probability of collision between patella and the medial femoral prosthesis 200 during postoperative knee flexion and extension, and reduce postoperative pain and the probability of loosening of the medial femoral prosthesis 200. Further, for example, the median sagittal plane is a mirror surface, and a medial femoral prosthesis applied to a medial compartment of the left knee joint can be obtained by mirroring the medial femoral prosthesis 200 of the right knee joint shown in Figure 8 to Figure 10.

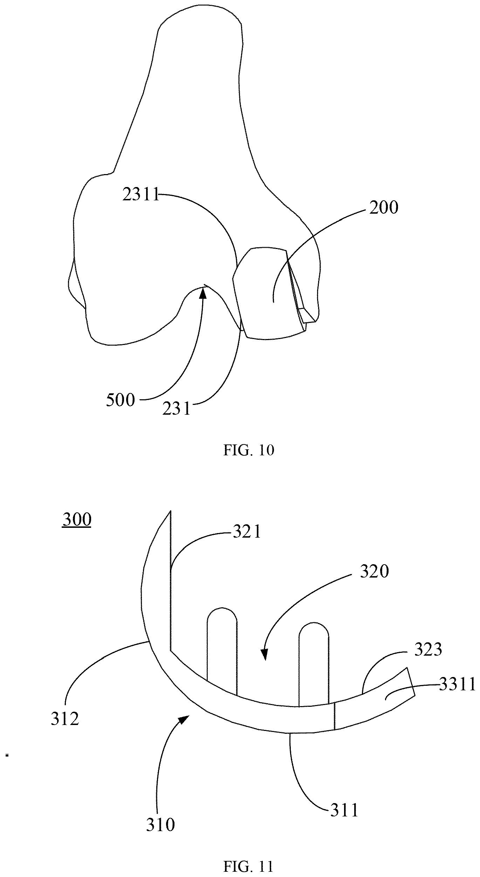

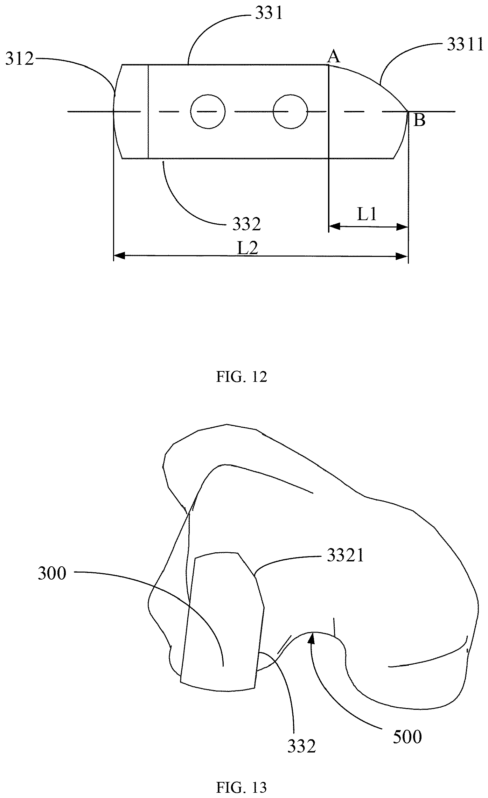

Referring to Figures 11-13, taking a lateral femoral prosthesis 300 of the right knee joint as an example, the side of the lateral femoral prosthesis 300 close to the knee joint center 500 is a medial surface 331, and the side of the medial femoral prosthesis 200 away from the knee joint center 500 is a lateral surface 332. A curved surface 3311 deviating from the direction of the knee joint center 500 is formed on the medial surface 331 of the lateral femoral prosthesis 300. Specifically, in the top view shown in Figure 12, the curved surface 3311 extends from a point A on a first half part of the medial surface 331 of the lateral femoral prosthesis 300 to a point B located at an anterior end of the lateral femoral prosthesis 300. Preferably, the point A is located at the first 35% to 45% of the medial surface 331, and more preferably, the point A is located at the first 40% of the medial surface 331. "Located at the first 35% to 45% of the medial surface 331" means that a distance L1 from the point A to the point B in an anterior-posterior direction accounts for 35% to 45% of a distance L2 from the most anterior end to the most posterior end of the lateral femoral prosthesis 300 in the anterior-posterior direction. The lateral femoral prosthesis 300 with the curved surface 3311 can reduce the probability of collision between patella and the lateral femoral prosthesis 300 during postoperative knee flexion and extension, and reduce postoperative pain and the probability of loosening of the lateral femoral prosthesis 300. Further, for example, the median sagittal plane is a mirror surface, and a lateral femoral prosthesis applied to a lateral compartment of the left knee joint can be obtained by mirroring the lateral femoral prosthesis 300 of the right knee joint shown in Figure 11 to Figure 13.

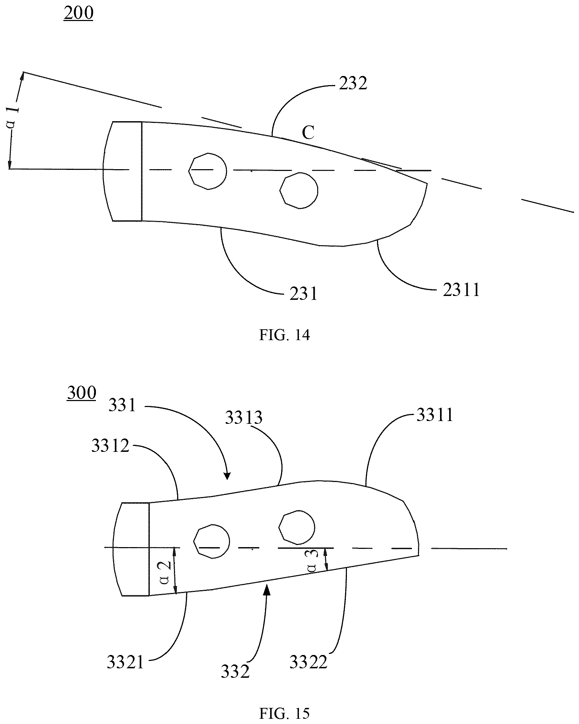

In one embodiment, the various embodiments of the present application also make further improvements to the medial surface 231 and the lateral surface 232 of the medial femoral prosthesis 200. Specifically, referring to Figure 14, taking the medial femoral prosthesis 200 of the right knee joint as an example, the lateral surface 232 and the medial surface 231 of the medial femoral prosthesis 200 are both curved surfaces curved toward the knee joint center. Optionally, an arc 2311 deviating from an extension direction of the medial surface 231 is further formed on the medial surface 231 of the medial femoral prosthesis 200, and the arc 2311 extends from a point located on a first half part of one side of the unicondylar femoral prosthesis 200 close to the knee joint center to a point located at an anterior end of the unicondylar femoral prosthesis 200. A positional relationship between the two points may be obtained with reference to the embodiment shown in Figure 9. Further, still referring to Figure 14, a projection of the lateral surface 232 on a horizontal plane forms an arc, and an angle α1 between a tangent at a midpoint C of the arc and the sagittal plane ranges from 15° to 30°, preferably 23° to 27°, more preferably 25°. When the angle α1 is within the above range, an anatomical shape of the medial femoral prosthesis 200 is better matched with the medial femoral condyle, which avoids the interference with medial ligament tissues of the knee joint caused by the overhang of the anterior end of the medial femoral prosthesis 200, and reduces the probability of postoperative pain. Further, for example, the median sagittal plane is a mirror surface, and a medial femoral prosthesis applied to a medial compartment of the left knee joint can be obtained by mirroring the medial femoral prosthesis 200 of the right knee joint shown in Figure 14.

In another embodiment, the exemplary embodiments of the present application also make further improvements to the medial surface 331 and the lateral surface 332 of the lateral femoral prosthesis 300. Specifically, referring to Figure 15, taking the lateral femoral prosthesis 300 of the right knee joint as an example, the lateral surface 332 of the lateral femoral prosthesis 300 includes a first straight surface 3321 and a second straight surface 3322 that are toward the knee joint center. The medial surface 331 includes a third straight surface 3312 and a fourth straight surface 3313 that are toward the knee joint center. Optionally, an arc 3311 deviating from an extension direction of the fourth straight surface 3313 is further formed on the medial surface 331 of the lateral femoral prosthesis 300, and the arc 3311 extends from a point located on a first half part of one side of the unicondylar femoral prosthesis 300 close to the knee joint center to a point located at an anterior end of the unicondylar femoral prosthesis 300. A positional relationship between the two points may be obtained with reference to the embodiment shown in Figure 12. The first straight surface 3321 and the third straight surface 3312 are parallel to each other, and an angle α2 between the first straight surface 3321 or the third straight surface 3312 and the sagittal plane ranges from 0° to 6°, preferably 3° to 5 °, more preferably 3°. The second straight surface 3322 and the fourth straight surface 3313 are parallel to each other, and an angle α3 between the second straight surface 3322 or the fourth straight surface 3313 and the sagittal plane ranges from 7° to 14°, preferably 7° to 11°, more preferably 9°. When the angle α2 and the angle α3 are within the above range, an anatomical shape of the lateral femoral prosthesis 300 is better matched with the lateral femoral condyle, which avoids the interference with lateral ligament tissues of the knee joint caused by the overhang of the anterior end of the lateral femoral prosthesis 300, and reduces the probability of postoperative pain. Further, for example, the median sagittal plane is a mirror surface, and a lateral femoral prosthesis applied to a lateral compartment of the left knee joint can be obtained by mirroring the lateral femoral prosthesis 300 of the right knee joint shown in Figure 15.

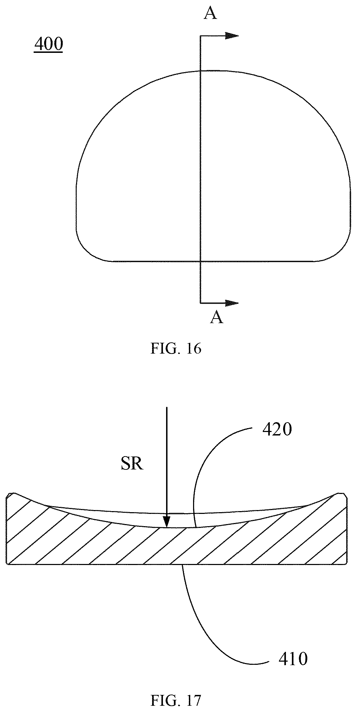

The embodiments of the present application further provide a tibial pad 400 for use with the unicondylar femoral prosthesis 100, 200, 300 according to any one of the above embodiments. Specifically, referring to Figures 16-17, the tibial pad 400 according to an embodiment includes a distal surface 410 and a proximal articular surface 420. The distal surface 410 is a flat surface, configured to connect with a tibia. The proximal articular surface 420 is configured to abut the distal articular surface 111 of the unicondylar femoral prosthesis 100. Further, the proximal articular surface 420 is a spherical surface, and a curvature radius SR of the proximal articular surface 420 is substantially the same as the first curvature radius Rd of the distal articular surface 111 with which it mates. Specifically, a ratio of the first curvature radius Rd to the curvature radius SR of the proximal articular surface 420 ranges from 0.95 to 1.

When the tibial pad 400 is in a state where the knee joint is straightened, that is, when the tibial pad 400 is in contact with the distal articular surface 111 of the unicondylar femoral prosthesis 100, due to the curvature radius SR of the proximal articular surface 420 being substantially the same as the curvature radius Rd of the distal articular surface 111 on the sagittal plane of 111 and a curvature radius Rc on the coronal plane, and the proximal articular surface 420 and the distal articular surface 111 can be highly matched. In this case, the posture of the human body is a posture where the knee joint is under greater pressure such as standing and walking. Therefore, heights of the tibial pad 400 and the unicondylar femoral prosthesis 100 can be matched, so that the tibial pad 400 and the unicondylar femoral prosthesis 100 can have better contact and friction performance, thereby reducing the wear of the unicondylar femoral prosthesis 100 and prolonging the service life of the unicondylar femoral prosthesis 100.

When the knee joint is in a flexed state, that is, when the tibial pad 400 is in contact with the posterior condyle articular surface 112 of the unicondylar femoral prosthesis 100, although the curvature radius SR of the proximal articular surface 420 is not substantially the same as a sagittal-plane curvature radius (i.e., the third curvature radius Rp) of the posterior condyle articular surface 112, the proximal articular surface 420 and the posterior condyle articular surface 112 can also be highly of matched on the coronal plane because the curvature radius SR of the proximal articular surface 420 is similar to a coronal-plane curvature radius (i.e., the third curvature radius Rc) of the posterior condyle articular surface 112. When the knee joint is in the flexed state, the human body is mostly sitting. In this case, the knee joint bears little pressure, and the height matching of the coronal plane is sufficient to ensure better contact friction performance, thereby reducing the wear of the unicondylar femoral prosthesis 100 and prolonging the service life of the unicondylar femoral prosthesis 100.

Technical features of the above embodiments may be combined randomly. To make descriptions brief, not all possible combinations of the technical features in the embodiments are described. Therefore, as long as there is no contradiction between the combinations of the technical features, they should all be considered as scopes disclosed in the specification.

The above embodiments only describe several implementations of the present application, and their description is specific and detailed, but cannot therefore be understood as a limitation on the patent scope of the present application. It should be noted that those of ordinary skill in the art may further make variations and improvements without departing from the conception of the present application, and these all fall within the protection scope of the present application. Therefore, the patent protection scope of the present application should be subject to the appended claims.

## Claims

1. A unicondylar femoral prosthesis, wherein the unicondylar femoral prosthesis comprises:
an osteotomy surface configured to connect with a femur; and
an articular surface configured to abut a tibial pad, the articular surface comprising:
a distal articular surface, the distal articular surface having a first curvature radius on a sagittal plane; and
a posterior condyle articular surface, the posterior condyle articular surface having a second curvature radius on the sagittal plane;
wherein a ratio of the first curvature radius to the second curvature radius ranges from 1.4 to 2.1, the articular surface has a third curvature radius on a coronal plane, and the third curvature radius is equal to the first curvature radius.

2. The unicondylar femoral prosthesis according to claim 1, wherein the osteotomy surface comprises a posterior condyle osteotomy surface and a distal osteotomy surface, the posterior condyle osteotomy surface is a flat surface, the distal osteotomy surface is a spherical surface, and the distal osteotomy surface is concentric with the distal articular surface.

3. The unicondylar femoral prosthesis according to claim 2, wherein a curvature radius of the distal osteotomy surface is 4 mm to 7 mm less than the first curvature radius.

4. The unicondylar femoral prosthesis according to claim 1, wherein a curved surface deviating from a direction of a knee joint center is formed on one side of the unicondylar femoral prosthesis close to the knee joint center.

5. The unicondylar femoral prosthesis according to claim 4, wherein the curved surface extends from a point located on a first half part of one side of the unicondylar femoral prosthesis close to the knee joint center to a point located at an anterior end of the unicondylar femoral prosthesis.

6. The unicondylar femoral prosthesis according to claim 1, wherein a transition angle between the distal articular surface and the posterior condyle articular surface ranges from 10° to 25°.

7. The unicondylar femoral prosthesis according to any one of claims 1 to 6, wherein the unicondylar femoral prosthesis is a medial femoral prosthesis configured to replace a medial compartment of a femur, and a first curvature radius of the medial femoral prosthesis ranges from 30 mm to 55 mm.

8. The unicondylar femoral prosthesis according to claim 7, wherein the medial femoral prosthesis has a lateral surface and a medial surface, and both the lateral surface and the medial surface comprise a curved surface curved toward the knee joint center.

9. The unicondylar femoral prosthesis according to claim 8, wherein a projection of the lateral surface on a horizontal plane forms an arc, and an angle between a tangent at a midpoint of the arc and the sagittal plane ranges from 15° to 30°.

10. The unicondylar femoral prosthesis according to any one of claims 1 to 6, wherein the unicondylar femoral prosthesis is a lateral femoral prosthesis configured to replace a lateral compartment of a femur, and a first curvature radius of the lateral femoral prosthesis ranges from 30 mm to 60 mm.

11. The unicondylar femoral prosthesis according to claim 10, wherein the lateral femoral prosthesis has a lateral surface and a medial surface, the lateral surface comprises a first straight surface and a second straight surface that are toward the knee joint center, and the medial surface comprises a third straight surface and a fourth straight surface that are toward the knee joint center.

12. The unicondylar femoral prosthesis according to claim 10, wherein the first straight surface is parallel to the third straight surface, and an angle between the first straight surface or the third straight surface and the sagittal plane ranges from 0° to 6°; and the second straight surface is parallel to the fourth straight surface, and an angle between the second straight surface or the fourth straight surface and the sagittal plane ranges from 7° to 14°.

13. A tibial pad for mating with the unicondylar femoral prosthesis according to any one of claims 1 to 12, wherein the tibial pad has a proximal articular surface abutting the distal articular surface, the proximal articular surface is a spherical surface, and a ratio of the first curvature radius to a curvature radius of the proximal articular surface ranges from 0.95 to 1.

14. A unicondylar replacement prosthesis, comprising the unicondylar femoral prosthesis according to any one of claims 1 to 12 and the tibial pad according to claim 13.
